# EUROPÄISCHE PATENTSCHRIFT

(11) **EP 2 229 401 B1**
(45) Veröffentlichungstag und Bekanntmachung des Hinweises auf die Patenterteilung: **21.08.2013**
(21) Anmeldenummer: 08871155.1
(22) Anmeldetag: 20.11.2008
(51) Int. Cl.: C07K 7/06, A61P 35/00

(54) **HERSTELLUNG UND VERWENDUNG ANTITUMORALER CYCLODEPSIPEPTIDE**
PRODUCTION AND USE OF ANTITUMORAL CYCLODEPSIPEPTIDES
PRODUCTION ET UTILISATION DE CYCLODEPSIPEPTIDE ANTITUMORAL

(30) Priorität: 18.01.2008 DE 102008005097
(43) Veröffentlichungstag der Anmeldung: 22.09.2010
(73) Patentinhaber: Helmholtz-Zentrum für Ozeanforschung Kiel (GEOMAR), 24148 Kiel (DE)
(72) Erfinder: IMHOFF, Johannes, 24211 Preetz (DE); YU, Zhiguo, 110016 Shenyang (CN); LANG, Gerhard, 1796 Gurwolf (CH); WIESE, Jutta, 23701 Eutin (DE); KALTHOFF, Holger, 24106 Kiel (DE); KLOSE, Stephanie, 38448 Wolfsburg (DE)
(74) Vertreter: UEXKÜLL & STOLBERG
(86) Internationale Anmeldenummer: PCT/DE2008/001915
(87) Internationale Veröffentlichungsnummer: WO 2009/089810

(56) Entgegenhaltungen:
- WO-A-2004/035613
- JIMENO JOSÉ ET AL: "Adding pharmacogenomics to the development of new marine-derived anticancer agents" JOURNAL OF TRANSLATIONAL MEDICINE, BIOMED CENTRAL, LONDON, GB, Bd. 4, Nr. 1, 9. Januar 2006 (2006-01-09), Seite 3, XP021009902 ISSN: 1479-5876
- YU ZHIGUO ET AL: "Scopularides A and B, cyclodepsipeptides from a marine sponge-derived fungus, Scopulariopsis brevicaulis." JOURNAL OF NATURAL PRODUCTS JUN 2008, Bd. 71, Nr. 6, Juni 2008 (2008-06), Seiten 1052-1054, XP002514970 ISSN: 1520-6025

## Beschreibung

Die Erfindung betrifft zyklische Peptide, die zur Herstellung von Medikamenten zur Behandlung von Krebserkrankungen geeignet sind. Die Erfindung betrifft außerdem ein Verfahren zur Produktion dieser Verbindungen, sowie deren Verwendung als Arzneimittel.

Viele Krebserkrankungen sind bislang nicht durch selektiv wirkende biologische Wirkstoffe therapierbar. Nach Angaben der World Health Organization (WHO) wurde weltweit im Jahr 2000 bei ca. 10 Millionen Menschen Krebs diagnostiziert, ca. 6 Millionen verstarben (World Cancer Report 2003. http://www.iarc.fr/IARCPress/pdfs/wcr/WorldCancerReport.pdf ). Nach Schätzung der WHO wird die Zahl der durch Krebserkrankungen hervorgerufenen Todesfälle bis 2030 auf ca. 11,5 Millionen pro Jahr ansteigen (Worlds Health Statistics - 2007. http://www.who.int/whosis/whostat2007_10highlights.pdf). Ungefähr ab dem Jahr 2010 werden Krebserkrankungen nach Infektionserkrankungen die Hauptursache für Todesfälle darstellen, gefolgt von ischämischen Herzerkrankungen, Apoplexie (Hirninfarkt) und HIV/Aids.

Peptide, die von Mikroorganismen produziert werden, zählen zu vielversprechenden Kandidaten für die Behandlung von Krebserkrankungen. Beispielsweise Actinomycin D, das von *Streptomyces parvulus* produziert wird, wird bereits als Medikament zur Behandlung von Krebs eingesetzt (BC Cancer Agency Cancer Drug Manual - 1994. http://www.bccancer.bc.ca/NR/rdonlyres/852A1FC3-5BD2-481B-BFAA-45ACBFA77FAC/22684/Dactinomycinmonograph_5APR07.pdf). Thiocoralin, ein Produkt von *Micromonspora marina*, hemmt das Wachstum von Tumorzellen (Romero, F. et al. "Thiocoraline, a New Depsipeptide with Antitumor Activity Produced by a Marine Micromonospora. I. Taxonomy, Fermentation, Isolation, and Biological Activities." J. Antibiot. 1997; 50 (9): 734-7). Das marine Pilzisolat *Exserohilum rostratum* produziert die zyklischen Dipeptide Rostratin A-D, die eine zytotoxische Wirkung aufweisen (Tan, R.X. "Isolation and Structure Assignments of Rostratins A-D, Cytotoxic Disulfides Produced by the Marine-Derived Fungus Exserohilum rostratum." J. Nat. Prod. 2004 Aug; 67(8): 1374-82).

Trotz der weiten Verbreitung der Gattung *Scopulariopsis,* auch im marinen Habitat, gibt es nur begrenzte Kenntnisse über natürliche Produkte aus diesen Organismen. Unseres Wissens ist das einzige bisher bekannte natürliche Produkt aus dieser Gattung ein Pyranol-Derivat, patentiert für seine antimykotische Aktivität (US 5,270,334).

Im Hinblick auf die große Anzahl von Menschen, die an Krebs erkrankt sind, die ungünstige Prognose für die Heilung bestimmter Krebsarten (z.B. Pankreas) aufgrund der schlechten Wirksamkeit bisheriger Medikamente, Nebenwirkungen und Resistenzentwicklung gegenüber eingesetzten Arzneimitteln besteht ein dringender Bedarf an neuen Krebsmedikamenten.

Damit liegt der vorliegenden Erfindung die Aufgabe zugrunde, weitere antitumoral wirkende Peptide bereitzustellen, sowie einen Weg zu ihrer Produktion aufzuzeigen.

Erfindungsgemäß wird diese Aufgabe durch die Verbindung mit den Merkmalen von Anspruch 1 gelöst. Die Unteransprüche geben vorteilhafte Ausführungen der Erfindung wieder.

Die Erfindung betrifft in einem ersten Aspekt eine Verbindung der allgemeinen Struktur 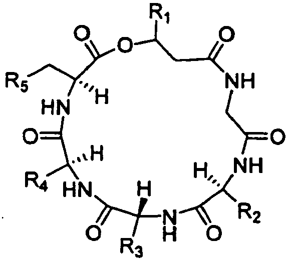 wobei R₁, R₂, R₃ und R₄ ausgewählt sind aus der Gruppe bestehend aus einem WasserstoffAtom (H) und einem unsubstituierten oder einem einen Substituenten aufweisenden C₁-C₂₀-Alkyl, und R₅ ein unsubstituierter oder ein einen Substituenten aufweisender Phenylrest ist, wobei das Alkyl und/oder der Phenylrest einen Substituenten ausgewählt aus der Gruppe bestehend aus einem Acylrest, einer Formyl-, Acetyl-, Trichloracetyl-, Fumaryl-, Maleyl-, Succinyl- oder Benzoylgruppe, einem Halogenrest, einer Aminoalkylgruppe, einer Hydroxylgruppe, einer Ethergruppe oder einer Carboxylgruppe aufweist.

In einer bevorzugten Ausführungsform ist die Verbindung eine Verbindung der Formel 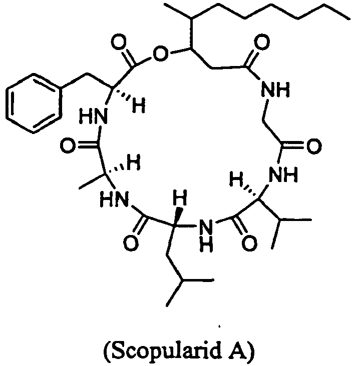 einschließlich dessen Diastereomere. In einer weiteren bevorzugten Ausführungsform ist die Verbindung eine Verbindung der Formel 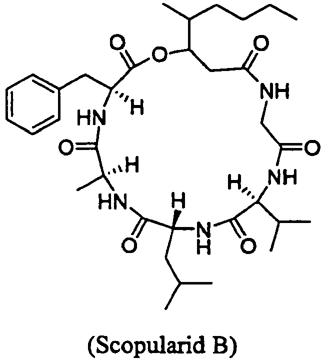 einschließlich dessen Diastereomere.

In einem weiteren Aspekt betrifft die Erfindung ein Verfahren zur Herstellung einer wie oben beschriebenen Verbindung, das durch die folgenden Schritte gekennzeichnet ist:
- Kultivieren eines Pilzes der Gattung *Scopulariopsis* auf an sich bekannte Weise, und
- Isolieren der Verbindung aus dem Kulturmedium und/oder dem Pilz. Der Pilz ist vorzugsweise *Scopulariopsis brevicaulis*.

In einem noch weiteren Aspekt betrifft die Erfindung eine wie oben beschriebene Verbindung zur Verwendung als Antitumormittel. Die Erfindung betrifft schließlich auch eine wie oben beschriebene Verbindung zur Verwendung in einem Verfahren zur Behandlung von und zur Vorbeugung vor Pankreaskrebs.

Innerhalb eines wissenschaftlichen Programms zur Isolierung von biologisch aktiven natürlichen Produkten aus marinen Mikroorganismen haben die Erfinder den Pilz *Scopulariopsis brevicaulis*, isoliert aus dem Meeresschwamm *Tethya aurantium*, untersucht.

Die erfindungsgemäß bevorzugten antitumoralen Peptide Scopularid A und Scopularid B wurden aus Kulturen des Pilzes *Scopulariopsis brevicaulis* durch präparative HPLC isoliert.

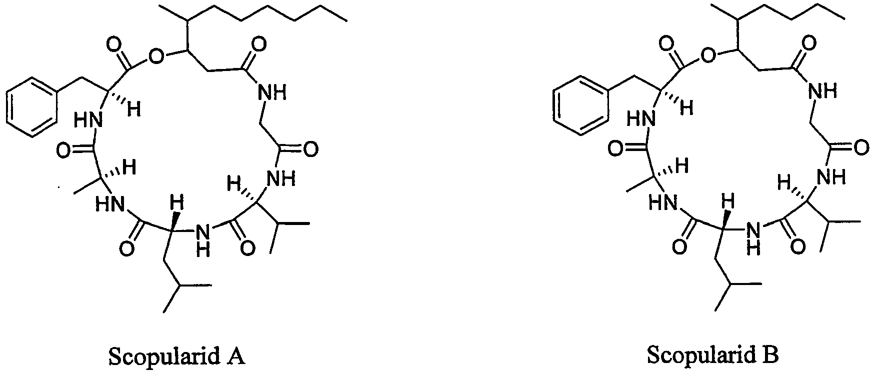

Die physikalischen Daten von Scopularid A und B sind in den beigefügten Tabellen 1 und 2 dargestellt, wobei Tabelle 1 die NMR-Daten (600 MHz, MeOD) von Scopularid A und Tabelle 2 die NMR-Daten (600 MHz, MeOD) von Scopularid B zeigt.

Bei der Untersuchung der antiproliferativen Wirkung gegen Tumorzellen wurde nachgewiesen, dass Scopularid B signifikante Hemmwirkungen gegen Tumorzelllinien zeigt, insbesondere gegen Pankreastumor-Zelllinie Colo357 (s. Beispiel 4).

Die Anzucht und die Identifizierung des Pilzes *Scopulariopsis brevicaulis*, so wie Aufreinigung von Scopularid A und Scopularid B aus Kulturen des Pilzes und die Bestimmung der antiproliferativen Wirkung gegen Tumorzellen werden in folgenden Beispielen beschrieben.

### 1) Anzucht einer Kultur des Pilzes Scopulariopsis brevicaulis

Die Anzucht der Pilzkulturen für die Gewinnung von Scopulariden erfolgte in 2-Liter-Erlenmeyerkolben, die mit je 700 ml Nährlösung folgender Zusammensetzung (modifiziert nach Wickerham, L.J. "Taxonomy of yeasts." US Dept. Technol. Bull. 1951; 1029: 1-56.) beschickt wurden: 3,0 g Hefeextrakt; 3,0 g Malzextrakt; 5,0 g Pepton; 10,0 g Glucose; 30,0 g NaCl; pH 6,8. Die Sterilisation der Nährlösung erfolgte durch Autoklavieren bei 121 °C/1 bar, 20 Minuten. Als Inokulum für die experimentelle Pilzkultur wurde von einer 14 Tage alten Vorkultur, die auf WM-Medium angezogen wurde, mit einer Impföse Zellmaterial in den Ansatz der Hauptkultur überführt. Die Inkubation der beimpften Nährlösung erfolgte über einen Zeitraum von 14 Tagen bei konstant 28°C in statischer Kultur im Dunkeln.

### 2) Identifizierung des Pilzes als Scopulariopsis brevicaulis

Die morphologischen Merkmale des Pilzes werden anhand der rasterelektronenmikroskopischen Untersuchungen ermittelt. Der Pilz zeigt einzelne Konidiophoren, die aus Hyphen hervorgehen. Die Hyphen sind unverzweigt, oder ein- bzw. zweimal vertikal unterteilt. Die konidiogenen Zellen sind zylindrisch, manchmal mit einer aufgequollenen Basis, und mit einer gleichmäßig weiten annellierende Zone variabler Länge. Die Konidien sind kugelförmig bis oval (4-6 x 6-7 µm) und weisen eine abgeflachte Basis sowie rauhe Erhebungen auf. Die genetische Identifizierung erfolgt anhand der 18S rDNA-Analyse. Die morphologischen Kennzeichen und die 18S rDNA Sequenz ergeben eine eindeutige Identifizierung des Pilzes als *Scopulariopsis brevicaulis*.

### 3) Aufreinigung von Scopularid A und Scopularid B aus Kulturen des Pilzes Scopulariopsis brevicaulis

Das Pilzmycel wird vom Kulturüberstand abgetrennt, zerkleinert und mit Aceton extrahiert. Der Acetonextrakt wird zur Trockne eingedampft, und der Rückstand wird nacheinander mit einem Methanol-Wasser-Gemisch (2:8) und mit n-Hexan gewaschen. Der verbleibende Rückstand wird einer Auftrennung durch präparative Hochleistungsflüssigchromatographie unterzogen:

| | |
|---|---|
| Trennsäule: | Phenomenex Luna C18, 21.2 x 250 mm, 5 µm |
| Lösungsmittel: | Wasser + 0.1% Ameisensäure und Acetonitril + 0.1 % Ameisen säure (30:70) |
| Flussrate: | 18 ml/min |

Die Substanzen Scopularid A und Scopularid B werden nach 13 bzw. 8 min eluiert. Nach Entfernen der Lösungsmittel durch Trocknen am Vakuum erhält man die erwünschten Reinsubstanzen als weiße Feststoffe.

### 4) Bestimmung der antiproliferativen Wirkung gegen Tumorzellen

Die Substanzen Scopularid A und Scopularid B zeigten signifikante Hemmwirkungen gegen Tumorzelllinien, insbesondere Pankreastumor-Zelllinien Colo357 und Panc89 sowie Darmtumorzelllinie HT29 (durchschnittlich zwischen 25-50 % Hemmung bei 10µg/ml). Diese Aktivität wurde mit dem Kristallviolett-Assay bestimmt (Siegmund, D. et al. "Death receptorinduced signalling pathways are differentially regulated by gamma interferon upstream of caspase 8 processing." Mol. Cell. Biol. 2005; 25: 6363-6379).

**Tabelle 1: Scopularid A**

| | | | | | |
|---|---|---|---|---|---|
| Farbloser feinkristalliner Feststoff. | | | | | |
| Schmelzpunkt: 229-230 °C | | | | | |
| Optische Aktivität: [α]25D -38° (c 0.5, MeOH) | | | | | |
| HRESIMS: m/z 672.4331 [M+H]+ (berechnet für C₃₆H₅₈N₅O₇ 672.4336) | | | | | |
| NMR-Daten (600MHz; in MeOH-d4) | | | | | |
| | | | | | |

| **Position** | | **δ_{C}, mult.** | **δ**_{H} **(*J* in Hz)** | **COSY** | **HMBC** |
|---|---|---|---|---|---|
| *Phe* | 1 | 173.6, qC | | | |
| | 2 | 55.3, CH | 4.79, m | 3a, 3b | 1, 3 |
| | 3 | 39.4, CH₂ | 3.18, dd (13.5, 7.5) | 2, 3b | 1, 2, 4, 5/9 |
| | | | 3.03, dd (13.5, 8.2) | 2, 3a | 1, 2, 4, 5/9 |
| | 4 | 137.8, qC | | | |
| | 5/9 | 130.3, CH | 7.24, m | 6/8 | 3, 7, 5/9 |
| | 6/8 | 129.6, CH | 7.28, m | 5/9 | 4, 6/8 |
| | 7 | 128.0, CH | 7.21, m | | 5/9 |
| *Ala* | 1 | 174.1, qC | | | |
| | 2 | 50.3, CH | 4.29, q (7.3) | 3 | 1, 3, *Leu*-1 |
| | 3 | 17.6, CH₃ | 1.27, d (7.3) | 2 | 1, 2 |
| *Leu* | 1 | 175.1, qC | | | |
| | 2 | 54.7, CH | 4.19, m | 3 | 1, 3, 4 |
| | 3 | 40.3, CH₂ | 1.58, m | 2, 4 | 1, 2, 4 |
| | 4 | 26.0, CH | 1.68, m | 3, 5, 6 | 2, 3, 5, 6 |
| | 5 | 23.1, CH₃ | 1.00, d (6.6) | 4 | 3, 4, 6 |
| | 6 | 22.3, CH₃ | 0.95, d (6.6) | 4 | 3, 4, 5 |
| *Val* | 1 | 173.4, qC | | | |
| | 2 | 59.9, CH | 4.20, m | 3 | 1, 3, 4, *Gly*-1 |
| | 3 | 31.0, CH | 2.19, m | 2, 4, 5 | 1, 2, 4, 5 |
| | 4 | 19.0, CH₃ | 0.98, d (6.7) | 3 | 2, 3, 5 |
| | 5 | 19.7, CH₃ | 0.97, d (6.7) | 3 | 2, 3, 4 |
| *Gly* | 1 | 171.9, qC | | | |
| | 2 | 44.0, CH₂ | 4.24, d (16.8) | | 1, *HMDA*-1 |
| | | | 3.53, d (16.8) | | 1, *HMDA*-1 |
| *HMDA*^{a} | 1 | 174.5, qC | | | |
| | 2 | 40.9, CH₂ | 2.42, m | 3 | 1, 3, 4 |
| | 3 | 78.6, CH | 4.77, m | 2, 4 | 2, 4, 4-Me |
| | 4 | 39.0, CH | 1.54, m | 3, 4-Me | |
| | 5 | 33.6, CH₂ | 1.22, m | | |
| | | | 0.95, m | | |
| | 6 | 30.6, CH₂ | 1.30, m | | |
| | 7 | 28.2, CH₂ | 1.29, m | | |
| | 8 | 33.0, CH₂ | 1.26, m | | |
| | 9 | 23.7, CH₂ | 1.31, m | 10 | |
| | 10 | 14.42, CH₃ | 0.91, t (7.2) | 9 | 9, 8 |
| | 4-Me | 14.41, CH₃ | 0.83, d (6.9) | 4 | 3 ,4, 5 |

| | | | | | |
|---|---|---|---|---|---|
| ^{a} HMDA: 3-Hydroxy-4-methyldecansäure | | | | | |

**Tabelle 2: Scopularid B**

| | | | | | |
|---|---|---|---|---|---|
| Weißer, amorpher Feststoff | | | | | |
| Optische Aktivität: [α]25D -43° (c 0.5, MeOH) | | | | | |
| HRESIMS: m/z 644.4007 [M+H]+ (berechnet für C₃₄H₅₄N₅O₇ 644.4023) | | | | | |
| NMR-Daten (600MHz; in MeOH-d4) | | | | | |
| | | | | | |

| **Position** | | **δ_{C}, mult.** | **δ_{H} (*J* in Hz)** | **COSY** | **HMBC** |
|---|---|---|---|---|---|
| *Phe* | 1 | 173.6, qC | | | |
| | 2 | 55.3, CH | 4.78, m | 3a, 3b | 1, 3 |
| | 3 | 39.4, CH₂ | 3.18, dd (13.5, 7.5) | 2, 3b | 1, 2, 4, 5/9 |
| | | | 3.03, dd (13.5, 8.2) | 2, 3a | 1, 2, 4, 5/9 |
| | 4 | 137.8, qC | | | |
| | 5/9 | 130.3, CH | 7.24, m | 6/8 | 3, 7, 5/9 |
| | 6/8 | 129.6, CH | 7.28, m | 5/9 | 4, 6/8 |
| | 7 | 128.0, CH | 7.21, m | | 5/9 |
| *Ala* | 1 | 174.1, qC | | | |
| | 2 | 50.3, CH | 4.29, q (7.3) | 3 | 1, 3, *Leu*-1 |
| | 3 | 17.6, CH₃ | 1.28, d (7.3) | 2 | 1, 2 |
| *Leu* | 1 | 175.1, qC | | | |
| | 2 | 54.7, CH | 4.19, m | 3 | 1, 3, 4 |
| | 3 | 40.4, CH₂ | 1.58, m | 2,4 | 1, 2, 4 |
| | 4 | 26.0, CH | 1.69, m | 3,5,6 | 2, 3, 5, 6 |
| | 5 | 23.1, CH₃ | 0.99, d (6.6) | 4 | 3, 4, 6 |
| | 6 | 22.3, CH₃ | 0.95, d (6.6) | 4 | 3, 4, 5 |
| *Val* | 1 | 173.4, qC | | | |
| | 2 | 59.9, CH | 4.20, m | 3 | 1, 3, 4, *Gly*-1 |
| | 3 | 31.0, CH | 2.18, m | 2, 4, 5 | 1, 2, 4, 5 |
| | 4 | 19.0, CH₃ | 0.98, d (6.7) | 3 | 2, 3, 5 |
| | 5 | 19.7, CH₃ | 0.97, d (6.7) | 3 | 2, 3, 4 |
| *Gly* | 1 | 171.9, qC | | | |
| | 2 | 44.0, CH₂ | 4.24, d (16.8) | | 1, *HMOA*-1 |
| | | | 3.53, d (16.8) | | 1, *HMOA*-1 |
| *HMOA^{a}* | 1 | 174.5, qC | | | |
| | 2 | 40.9, CH₂ | 2.41, m | 3 | 1, 3 ,4 |
| | 3 | 78.6, CH | 4.77, m | 2, 4 | 2, 4, 4-Me |
| | 4 | 39.0, CH | 1.54, m | 3, 4-Me | |
| | 5 | 33.3, CH₂ | 1.22, m | | |
| | | | 0.96, m | | |
| | 6 | 30.5, CH₂ | 1.32, m | 6b | |
| | | | 1.32, m | 6a | |
| | 7 | 23.8, CH₂ | 1.16, m | 8 | |
| | 8 | 14.38, CH₃ | 0.90, t (7.2) | 7 | 6, 7 |
| | 4-Me | 14.41, CH₃ | 0.83, d (6.9) | 4 | 3, 4 ,5 |

| | | | | | |
|---|---|---|---|---|---|
| ^{a} HMOA: 3-Hydroxy-4-methyloctansäure | | | | | |

## Patentansprüche

1. Verbindung der allgemeinen Struktur 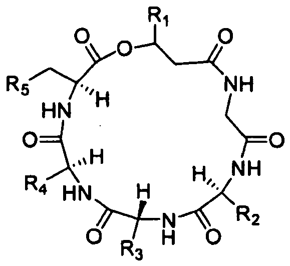 wobei
R₁, R₂, R₃ und R₄ ausgewählt sind aus der Gruppe bestehend aus einem Wasserstoff-Atom (H) und einem unsubstituierten oder einem einen Substituenten aufweisenden C₁-C₂₀-Alkyl,
und
R₅ ein unsubstituierter oder ein einen Substituenten aufweisender Phenylrest ist,
wobei das Alkyl und/oder der Phenylrest einen Substituenten ausgewählt aus der Gruppe bestehend aus einem Acylrest, einer Formyl-, Acetyl-, Trichloracetyl-, Fumaryl-, Maleyl-, Succinyl- oder Benzoylgruppe, einem Halogenrest, einer Aminoalkylgruppe, einer Hydroxylgruppe, einer Ethergruppe oder einer Carboxylgruppe aufweist.

2. Verbindung nach Anspruch 1 mit der Formel 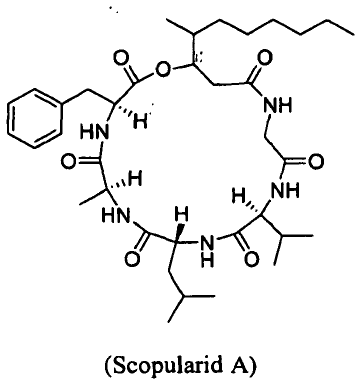 einschließlich dessen Diastereomere.

3. Verbindung nach Anspruch 1 mit der Formel 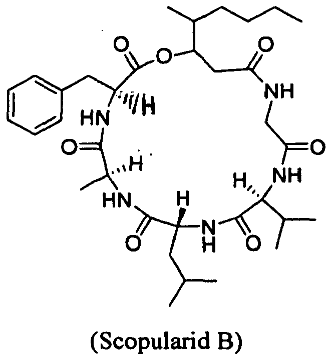 einschließlich dessen Diastereomere.

4. Verfahren zur Herstellung einer Verbindung nach einem der vorhergehenden Ansprüche, **gekennzeichnet durch** die Schritte:
- Kultivieren eines Pilzes der Gattung *Scopulariopsis* auf an sich bekannte Weise, und
- Isolieren der Verbindung aus dem Kulturmedium und/oder dem Pilz.

5. Verfahren nach Anspruch 4, **dadurch gekennzeichnet, dass** der Pilz *Scopulariopsis brevicaulis* ist.

6. Verbindung nach einem der Ansprüche 1 bis 3 zur Verwendung als Antitumormittel.

7. Verbindung nach einem der Ansprüche 1 bis 3 zur Verwendung in einem Verfahren zur Behandlung von und zur Vorbeugung vor Pankreaskrebs.

## Claims

1. Compound of the general structure 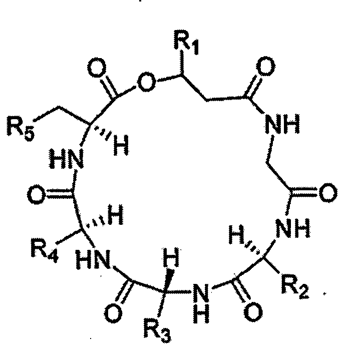 wherein
R₁, R₂, R₃, and R₄ are selected from the group consisting of a hydrogen atom (H) and an unsubstituted or substituted C₁ to C₂₀ alkyl,
and
R₅ is an unsubstituted or substituted phenyl residue,
wherein the alkyl and/or the phenyl residue comprises a substituent selected from the group consisting of an acyl residue, a formyl, acetyl, tricloroacetyl, fumaryl, maleyl, succinyl or benzoyl group, a halogen residue, an amino alkyl group, a hydroxyl group, an ether group, or a carboxyl group.

2. Compound according to claim 1 having the formula 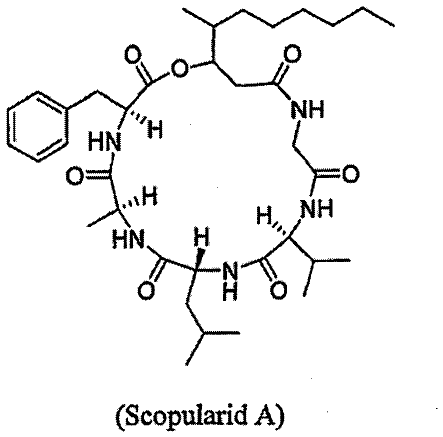 including its diastereomers.

3. Compound according to claim 1 having the formula 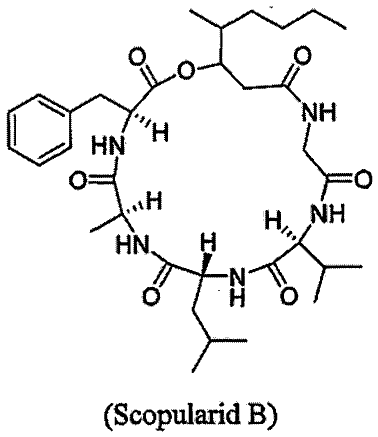 including its diastereomers.

4. Process for the production of a compound according to any one of the preceding claims, comprising
- culturing a fungus of the genus *Scopulariopsis* according to conventional measures, and
- isolating the compound from the culture medium and/or the fungus.

5. Process according to claim 4, wherein the fungus is *Scopulariopsis brevicaulis*.

6. Compound according to any one of claims 1 to 3 for use as an anti-tumor agent.

7. Compound according to any one of claims 1 to 3 for use in a method of treating and preventing pancreatic cancer.

## Revendications

1. Composé de structure générale 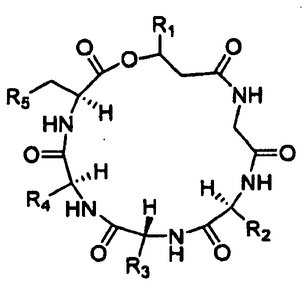 où
R₁, R₂, R₃ et R₄ sont choisis dans le groupe consistant en un atome d'hydrogène (H) et un alkyle en C₁-C₂₀ non substitué ou présentant un substituant,
et
R₅ est un radical phényle non substitué ou présentant un substituant,
où l'alkyle et/ou le radical phényle présentent un substituant choisi dans le groupe consistant en un radical acyle, un groupe formyle, acétyle, trichloracétyle, fumaryle, maléyle, succinyle ou benzoyle, un radical halogéno, un groupe aminoalkyle, un groupe hydroxyle, un groupe éther ou un groupe carboxyle.

2. Composé selon la revendication 1, avec la formule 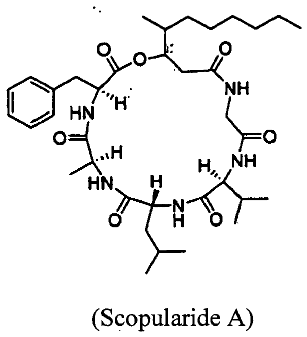 y compris ses diastéréoisomères.

3. Composé selon la revendication 1, avec la formule 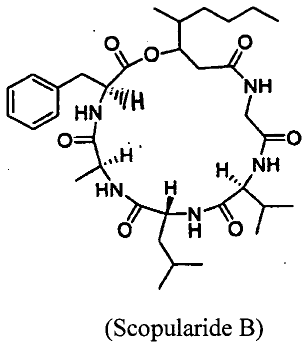 y compris ses diastéréoisomères.

4. Procédé de préparation d'un composé selon l'une des revendications précédentes, **caractérisé par** les étapes de :
- culture d'un champignon du genre *Scopulariopsis* d'une manière connue en soi, et
- isolement du composé du milieu de culture et/ou du champignon.

5. Procédé selon la revendication 4, **caractérisé en ce que** le champignon est le *Scopulariopsis brevicaulis*.

6. Composé selon l'une des revendications 1 à 3 pour une utilisation en tant qu'agent anti-tumoral.

7. Composé selon l'une des revendications 1 à 3 pour une utilisation dans un procédé de traitement et de prévention du cancer du pancréas.
